# EUROPEAN PATENT SPECIFICATION

(11) **EP 3 197 475 B1**
(45) Date of publication and mention of the grant of the patent: **18.03.2020**
(21) Application number: 15775402.9
(22) Date of filing: 21.09.2015
(51) Int. Cl.: A61K 31/683, A61K 38/01, A61K 9/00, A61K 9/06, A61K 9/12, A61K 31/7032, A61K 36/63, A61P 27/16, A61P 31/02, A61P 1/02

(54) **COMPOSITIONS USEFUL FOR THE PREVENTION AND/OR TREATMENT OF INFECTIONS AND INFLAMMATIONS**
ZUSAMMENSETZUNGEN UND VERFAHREN ZUR VORBEUGUNG UND/ODER BEHANDLUNG VON INFEKTIONEN UND ENTZÜNDUNGEN
COMPOSITIONS UTILES POUR LA PRÉVENTION ET/OU LE TRAITEMENT D'INFECTIONS ET D'INFLAMMATIONS

(30) Priority: 24.09.2014 IT MI20141660
(43) Date of publication of application: 02.08.2017
(73) Proprietor: Tower S.r.l. u.s., 10128 Torino (IT); Bict Srl, 26900 Lodi (LO) (IT)
(72) Inventor: CANDIOLI CRAVERO, Luca, 10092 Beinasco (TO) (IT); BRUNI, Natascia, 10092 Beinasco (TO) (IT); PANETTO, Enrico, 10092 Beinasco (TO) (IT); MAZZEI, Emma, 26900 Lodi (LO) (IT); RAPACIOLI, Silvia, 26900 Lodi (LO) (IT); VERGA, Roberto, 26900 Lodi (LO) (IT)
(74) Representative: Bianchetti Bracco Minoja S.r.l.
(86) International application number: PCT/EP2015/071548
(87) International publication number: WO 2016/046108

(56) References cited:
- WO-A1-02/38575
- WO-A1-2004/035071
- JP-B2- 3 746 081
- ROSANNA D.I. PAOLA ET AL: "Effects of verbascoside, biotechnologically purified by Syringa vulgaris plant cell cultures, in a rodent model of periodontitis", JOURNAL OF PHARMACY AND PHARMACOLOGY, vol. 63, no. 5, 1 April 2011 (2011-04-01), pages 707-717, XP055188376, ISSN: 0022-3573, DOI: 10.1111/j.2042-7158.2011.01262.x

## Description

The present invention relates to compositions comprising a lactoferrin hydrolysate and verbascoside or extracts containing it, and optionally glycerophosphoinositol or salts thereof.

The compositions according to the invention are useful for the prevention and/or treatment of dermatological, otological and stomatological inflammations and infections, in particular in the veterinary sphere. The compositions according to the invention can also be employed in the human pharmaceutical, animal feed, nutraceutical and cosmetic fields, and can be used in medical devices.

### STATE OF THE ART

Ear, skin and stomatological disorders in humans and animals, especially pets such as dogs and cats, present therapeutic problems that are not easy to solve. Said disorders are mainly due to infections supported by bacteria, fungi or parasites, and conventional pharmacological treatments based on antibiotics, antifungals or pesticides, possibly combined with anti-inflammatories, analgesics or other medicaments, do not always enable the disorder to be treated satisfactorily.

The primary causes of otitis externa, which has a prevalence of 15-20% in dogs and 6-7% in cats, are allergies or hypersensitivity (atopy, allergic reactions to food, allergic dermatitis caused by fleas), foreign bodies with subsequent aspecific bacterial superinfection, bacterial infection (*Bacillus* spp, *Pseudomonas* spp, *E. coli, Enterococcus* spp, *Proteus* spp, *Staphylococcus intermedius* and *Staphylococcus aureus, Streptococcus canis* and *Streptococcus* spp, *Klebsiella* spp), and yeast infection (*Candida* spp, *Malassezia pachydermatis,* and more rarely *M. furfur, M. globosa* and *M. sympodialis*).

Otitis is usually treated by cleaning of the auditory meatus and topical treatment, followed by systemic treatment if necessary. Products based on glucocorticoids (hydrocortisone, prednisolone, triamcinolone, betamethasone, dexamethasone or fluocinolone), antibiotics (neomycin, chloramphenicol, gentamicin, amikacin or ciclosporin) and antifungals (ketoconazole, miconazole or amphotericin B2) in an aqueous or oily carrier are normally used for topical treatment.

Disinfectants such as povidone-iodine and chlorhexidine are also used.

If mites are present an antiparasitic such as amitraz, fipronil or ivermectin is used.

For systemic treatment of advanced otitis, antibiotics such as trimethoprim-sulphasalazine, clindamycin, cephalexin and enrofloxacin are used (Jacobson, Tydskr. S .Afr. vet.Ver., 73(4): 162-170, 2002).

Microbial skin diseases, which are more frequent in dogs than cats, include superficial bacterial dermatitis, wherein the infection does not go beyond the basal membrane (intertrigo, bacterial folliculitis, mucocutaneous pyodermitis and bacterial overgrowth syndrome), and deep bacterial dermatitis (furunculosis, cellulitis), wherein the infection also involves the dermis and/or the subcutaneous tissue.

A form of infection which is frequent in dogs is "pyotraumatic dermatitis", also known as "hot spot".

The bacterial strains most frequently encountered in these infections are *Staphylococcus intermedius,* and *Staphylococcus spp, Escherichia coli, Proteus mirabilis, Pseudomonas aeruginosa* and *Pseudomonas* spp, *Bacillus* spp and *Streptococcus* spp.

Other micro-organisms commonly found on the skin are yeasts of the genus Malassezia (especially *Malassezia pachydermatis*) and Candida, which are frequently considered to be co-pathogens in bacterial dermatitis.

In cats, the strains most frequently isolated in bacterial dermatitis are *Staphylococcus intermedius, Staphylococcus aureus* and *Staphylococcus simulans.* Yeasts of the genus Malassezia are also frequently found in this animal species.

Bacterial dermatitis is typically treated with oral antibiotics. Treatment of superficial and deep pyodermitis requires systemic administration of antimicrobials, and topical application of antibacterial agents. Amoxicillin/clavulanic acid and cephalosporins (cephalexin, cefadroxil and cephalothin) are often used to treat skin infections (Sudhakara Reddy et al., Hindawi Publishing Corporation ISRN Veterinary Science, 2014).

An additional treatment includes baths with an antimicrobial shampoo 3 times a week. The antimicrobial agents most commonly used include benzoyl peroxide, chlorhexidine, iodine and triclosan.

Additional topical treatment comprises the application of fatty ointments, for example based on mupirocin (Dowling, Can Vet J 37, November 1996).

Finally, as regards stomatitis, which is normally caused by the accumulation of bacterial plaque on the teeth and periodontium, the bacterial species most commonly found (76%) in periodontal disease are *Porphyromonas denticanis, P. gulae* and *P. salivae,* which are Gram-negative anaerobic bacteria. In addition to said three species, hundreds of other bacterial species can be found in the saliva of dogs and cats, whether healthy or suffering from gingivostomatitis.

The treatment involves products based on chlorhexidine, essential oils, triclosan, fluorides, oxygenating agents, quaternary ammonium compounds, enzymes and amino-alcohol substitutes ("Periodontal Disease and Diet in Domestic Pets", Gorrel, American Society for Nutritional Sciences, 1998) or the administration of corticosteroids or non-steroidal anti-inflammatory drugs (Yi-Ping Hung, et al., Vet J. Jun 10, 2014) to reduce the inflammation, possibly combined with antibiotics (Corbee et al., J Anim Physiol Anim Nutr (Berl). Aug; 96(4):671-80, 2012).

It usually gives short-term results, but the long-term results are unsatisfactory in the absence of surgery. In the case of chronic stomatitis, oral cyclosporin is administered with the aim of reducing the gum inflammation, often after surgery (Lommer, J Vet Dent. Spring; 30(1):8-17, 2013).

The need is therefore felt for new treatments which are more effective than the conventional treatments, involve fewer toxic or side effects, are convenient to use, and are applicable to different animal species and different organs, tissues and mucous membranes.

This need has been met by combining a lactoferrin hydrolysate with glycerophosphorylinositol or salts thereof and/or verbascoside or extracts containing it.

The use of lactoferrin hydrolysates to treat joint, cartilage and skeletal disorders was disclosed in WO 2007/043900. Topical formulations of lactoferrin are disclosed in WO 2012/153301 and EP 2298338. US 5834424 discloses the use of lactoferrin to inhibit the penetration of *S. pyogenes* and *S. aureus* into host cells.

EP 1581044 discloses dental sticks for pets which can contain lactoferrin among numerous other ingredients. JP 3 746081 B2 discloses the veterinary use of hydrolysed lactoferrin for the topical treatment of atopic dermatitis and otitis externa.

Verbascoside, a caffeic acid glycoside present in various plants belonging to the *Oleaceae* group, possesses antibacterial, anti-inflammatory, immunomodulating, antitumoral, hypertensive, anti-hepatotoxic and painkilling activity. WO 2004/035071 A1 discloses the use of verbascoside for the treatment of ear disorders, e.g. otalgia, otitis externa and otomycosis. Di Paola et al (Journal of Pharmacy and Pharmacology, vol. 63, no. 5, 2011, pages 707-717) reports the use of a verbascoside containing extract for the treatment of periodontitis in a rodent model.

Glycerophosphoinositol, a metabolite of phosphoinositide, has been studied as an immune response modulator, and proposed for the treatment of septic shock (WO 20142053642). WO 02/38575 A1 teaches the use of glycerophosphoinositol salts (Ca, Zn, Na, K, arginine, lysine or choline salt) for human or veterinary application in the treatment of atopic dermatitis or gingival bacterial infections.

### Description of the invention

It has now been found that the combination of lactoferrin hydrolysates with verbascoside or extracts containing it and optionally glycerophosphoinositol presents synergic effects which can be conveniently exploited to treat various types of infection in the veterinary field, especially in the treatment of ear, skin and stomatological disorders.

The subject of the invention is therefore compositions containing a lactoferrin hydrolysate and verbascoside or extracts containing it and optionally glycerophosphoinositol.

The invention also relates to said compositions for use in the treatment of erythematous and ceruminous otitis externa in dogs and cats, dermatological treatment of skin infections of microbial origin in dogs and cats, and stomatological treatment of inflammations of the oral cavity, especially those affecting the gums (gingivitis and periodontitis), in dogs and cats.

The compositions according to the invention may contain a lactoferrin hydrolysate and verbascoside or extracts containing it, or a lactoferrin hydrolysate, glycerophosphorylinositol or salts thereof, and verbascoside or extracts containing it.

Compositions containing all three active ingredients (lactoferrin hydrolysate, glycerophosphoinositol and salts thereof and verbascoside) are preferred. Compositions only containing verbascoside and lactoferrin hydrolysate are equally preferred.

The compositions according to the invention will be formulated with suitable excipients in forms suitable for topical or oral administration.

Examples of suitable forms include solid forms (such as tablets, pills, rigid and soft capsules, powders, granulates and pastes for oral use), semisolid forms (such as creams, ointments, gels, fatty ointments, lubricants, pastes, emulsions and microemulsions and pastes for topical use), and liquid forms (such as syrups, shampoos, lotions, ampoules and drops).

In the topical formulations, the concentration by weight of the individual active ingredients falls within the following intervals:
- Lactoferrin hydrolysate: 0.0005-15%;
- Glycerophosphoinositol or salts thereof (solution containing at least 4% L-α-glycero-phospho-D-myo-inositol choline): 0.0020% -10%;
- Verbascoside: 0.0020% -15%.

The oral formulations will be suitable for daily administration of the active ingredients at doses falling within the following intervals per approximately ten kg of weight of the animal treated:
- Lactoferrin hydrolysate: 0.005 mg to 5 grams;
- Glycerophosphoinositol: 0.005 mg to 5 grams;
- Verbascoside: 0.005 mg to 5 grams.

Lactoferrin hydrolysate is known and available on the market or can be prepared by known methods, for example by treating lactoferrin with proteolytic enzymes. An example of lactoferrin hydrolysate which is well known, has been thoroughly studied and is available on the market is lactoferricin (Wakabayashi H, Takase M, Tomita M. Curr Pharm Des. 2003;9(16):1277-87; Dairy Science & Technology 94: 181-193. 2013; Eliassen LT, et al., Anticancer Res. 2002 Sep-Oct; 22(5):2703-10.

Verbascoside can be present as such or in the form of an extract of *Olea europaea, Syringa vulgaris* or other genera, species and varieties or cultivars containing them. Said ingredients are also available on the market.

Glycerophosphoinositol or the salts thereof are known and available on the market. The lysine salt is preferred.

The topical formulations according to the invention will be administered two or three times a day, in quantities ranging from 0.5 to 10-20 ml or more, depending on the surface area to be treated.

In the case of oral administration, the dose will depend on a number of factors such as the animal's weight and the type and severity of the disorder, and will be determined by the veterinary surgeon case by case. Approximately, however, the doses administered will be 0.5 mg/kg/day to 400 mg/kg/day of lactoferrin hydrolysate, 1 mg/kg/day to 400 mg/kg/day of verbascoside or the equivalent of an extract thereof, and 1 mg/kg/day to 400 mg/kg/day of glycerophosphoinositol or the equivalent of a salt thereof.

The antimicrobial activity of the active ingredients of the composition according to the invention has been evaluated on *Malassezia pachydermatis* DSM 6172, *Staphylococcus intermedius* ATCC 29663, *Pseudomonas aeruginosa* ATCC 27853, *Staphylococcus aureus* ATCC 29213, *Escherichia coli* ATCC 25922 and *Malassezia furfur* DSM 6170 using *in vitro* susceptibility tests with the antimicrobial dilution method (Clinical and Laboratory Standards Institute -CLSI- protocols), with which it was possible to determine the MIC (Minimum Inhibitory Concentration) for each compound.

For all the tests performed, there was a positive control of antifungal activity using fluconazole (for Malassezia) and ceftriaxone (for the bacterial strains) and a negative control (absence of the compounds) wherein correct microbial growth was evaluated.

The antimicrobial activity of the combinations of the various compounds towards the micro-organisms against which the individual compounds had proved active was also evaluated.

The results demonstrated the synergy of the ingredients of the composition according to the invention.

The antimicrobial activity of lactoferrin (Lf), lactoferrin hydrolysate (Lf hydro), extract of *Olea europaea* and derivatives thereof (verbascoside) was evaluated on *S. aureus* ATCC 29213.

| | **Lf (g/L)** | **Lf hydro (g/L)** | **Verbascoside (g/L)** |
|---|---|---|---|
| *S. aureus* | 20.00 | 2.00 | 3.0 |

The antimicrobial activity of the various combinations of compounds was also determined:

| | **LF + verbascoside** | **LF hydro + verbascoside** |
|---|---|---|
| *S. aureus* | SYN¹ | SYN² |

Key: SYN = synergy; the type of interaction was established by calculating FIC_{index} (MIC_{A in combination}/MIC_{A} +
MIC_{B in combination}/MIC_{B}): 1 -> FIC_{index}= 0.3; 2 -> FIC_{index}= 0.5
The experimental data obtained demonstrate the synergic action of the compounds when combined with one another.

Examples of formulations according to the invention, and the results of an *in vivo* trial, are set out below. Glycerophosphoinositol is present in the formulations as a 4% aqueous solution. The *Syringa vulgaris* extract has a verbascoside content of 10% by weight.

### Example 1: ear gel

| | |
|---|---|
| Glycerin | 11.49 g |
| Dipropylene glycol | 70.5 g |
| Propylene glycol | 10.3334 g |
| 20% chlorhexidine digluconate | 0.25 g |
| Glycolic extract of propolis | 0.5 g |
| Lavender essence | 0.5 g |
| Butyl hydroxytoluene | 0.01 g |
| Pharmaceutical lactic acid | 2.6666 g |
| Glycolic extract of aloe | 0.5 g |
| Salicylic acid | 0.25 g |
| Technical oleic acid | 3 g |
| Lactoferrin hydrolysate | 2.4 g |
| *Syringa vulgaris* extract | 2.2 g |
| Glycerophosphoinositol lysine salt | 1.6 g |

### Example 2: foam for external use

| | |
|---|---|
| 18b-glycyrrhetic acid | 0.4 g |
| Purified water | 62.56 g |
| Cocoyl diethanolamide | 2 g |
| Dipropylene glycol | 20 g |
| Polysorbate 20 | 3 g |
| Hydrogenated castor oil (40)OE | 5 g |
| Melaleuca essence | 3 g |
| Disodium EDTA | 0.02 g |
| 20% chlorhexidine digluconate | 2.5 g |
| Vitamin F ethyl ester | 1 g |
| Butyl hydroxytoluene | 0.02 g |
| Glycolic extract of aloe | 0.5 g |
| Lactoferrin hydrolysate | 1.8 g |
| *Syringa vulgaris extract* | 2.6 g |
| Glycerophosphoinositol lysine salt | 2 g |

### Example 3: shampoo for external use

| | |
|---|---|
| Purified water | 43.69 g |
| Cocoyl diethanolamide | 3 g |
| Cocamidopropyl betaine | 5 g |
| Propylene glycol | 5 g |
| Imidazolidinyl urea | 0.2 g |
| Lauryl ether sodium sulphate (Texapon NSO UP) | 30 g |
| Methyl + methylchloro-isothiazolinone | 0.1 g |
| PEG-7-Glyceryl Cocoate | 4 g |
| Undecylenic polyglycol | 2 g |
| Polysorbate 20 | 2 g |
| Tricosolfan | 4 g |
| Lavender essence | 0.5 g |
| Butyl hydroxytoluene | 0.01 g |
| Salicylic acid | 0.5 g |
| Lactoferrin hydrolysate | 1.6 g |
| *Syringa vulgaris* extract | 2.9 g |
| Glycerophosphoinositol lysine salt | 2.3 g |

### Example 4: mousse for external use

| | |
|---|---|
| Citric acid monohydrate | 0.37 g |
| Purified water | 86.94 g |
| Cocamidopropyl betaine | 3 g |
| Dipropylene glycol | 0.5 g |
| Imidazolidinyl urea | 0.2 g |
| Methyl + methylchloro-isothiazolinone | 0.1 g |
| Undecylenic polyglycol | 1 g |
| Disodium phosphate dodecahydrate | 5.89 g |
| Vitamin E acetate | 0.1 g |
| Hydrogenated castor oil (40)OE | 1 g |
| Vitamin F ethyl ester | 0.1 g |
| Lavender essence | 0.3 g |
| Salicylic acid | 0.5 g |
| Lactoferrin hydrolysate | 0.8 g |
| *Syringa vulgaris* extract | 3.1 g |
| Glycerophosphoinositol lysine salt | 2.5 g |

### Example 5: cream for external use

| | |
|---|---|
| Glycerin | 2 g |
| Cetyl stearyl alcohol | 3.6 g |
| Cetyl stearyl alcohol (12) OE | 2 g |
| Sodium cetyl stearyl sulphate | 0.4 g |
| Isopropyl myristate | 3 g |
| Sodium polyacrylate | 0.2 g |
| Lactic acid | 0.1 g |
| BHT | 0.02 g |
| Methyl-methylchloro-isothiazolinone | 0.1 g |
| Imidazolidinyl urea | 0.3 g |
| Lavender essence | 0.1 g |
| Purified water | 88.18 g |
| Lactoferrin hydrolysate | 0.5 g |
| *Syringa vulgaris* extract | 3.1 g |
| Glycerophosphoinositol lysine salt | 1.7 g |

### Example 6: stomatological gel

| | |
|---|---|
| Octanol | 10 g |
| Purified water | 26.78 g |
| Butyl hydroxytoluene | 0.02 g |
| Dipropylene glycol | 60 g |
| Hydroxypropylcellulose (Klucel MF) | 2 g |
| Imidazolidinyl urea | 0.2 g |
| Perfume: Victory 6B/650 | 1 g |
| Lactoferrin hydrolysate | 2.4 g |
| *Syringa vulgaris* extract | 0.9 g |
| Glycerophosphoinositol lysine salt | 1.5 g |

### Example 7: Clinical trial on 10 dogs suffering from ceruminous otitis externa

4 to 8 drops per ear of the formulation described in example 1 were administered twice a day to the animals studied (one drop being equivalent to about 0.05 ml).

The animals were selected according to good clinical practice (http://www.ema.europa.eu/docs/en_GB/document_library/Scientific_guideline/2009/10/ WC500004343.pdf) and only included in the study when the owner's informed consent had been obtained. 10 dogs suffering from recurrent erythematous and ceruminous otitis were selected.

Dogs of different breeds and genders, aged between 2 and 10 years, with the following characteristics, were included in the study:
1. More than three episodes of otitis in the last 18 months;
2. Otoscopic examination compatible with erythematous and ceruminous otitis;
3. Cytological examination compatible with overgrowth of bacteria (number of bacteria > 10 to 40/HPF) and/or Malassezia (number of yeasts > 10 to 40/HPF), presence of keratinocytes and absence of neutrophil granulocytes and macrophages;
4. In the event of severe pain and handling difficulty, the dog was given 1 mg/kg of oral prednisolone once a day for three days, and its inclusion in the study was reconsidered after an examination 48 hours after discontinuance of the treatment;
5. The owner's agreement to take the animal for check-ups on the established dates.

Dogs that had received systemic or topical antibiotic or antifungal ear treatments with ear cleansers in the last 7 days were excluded from the trial. Dogs suffering from otitis caused by parasites (otodectic mange, demodectic mange), foreign bodies, tumours (e.g. polyps blocking the meatus), chronic purulent idiopathic hyperplastic polypoid otitis, and/or otitis media, were also excluded.

At the time of selection (VS) the dogs presented mono- or bilateral otitis and met the inclusion criteria. An otoscopic and cytological examination of the cerumen was conducted. If the otoscopic examination and cytological results were compatible with bacterial overgrowth and/or Malassezia, a bacteriological test was conducted, and the selected animal was included in the study (inclusion visit VI):
The following tests were conducted at the inclusion visit (VI):
Evaluation of the condition of the auricle and the external auditory meatus;
Cytological test of cerumen;
Sampling of cerumen for bacteriological culture test;
Completion of modified CADESI form and VAS.

The examinations were repeated with the same data collection pattern every week for three weeks (V7, V15, V21). Samples for the bacteriological culture test were only taken at VI and VF.

Cerumen samples were taken from the external auditory meatus with a cotton bud. The samples collected were immediately seeded on plates of Columbia Agar Base (with the addition of 5% sheep blood) and incubated at +37°C for 24-48 h. The colonies which, due to their morphology, Gram and catalase, appeared to belong to the genus Staphylococcus spp., were then seeded on Mannitol Salt Agar (Oxoid®) for selective growth of staphylococci, and on a chromogenic medium specific for methicillin-resistant strains (MRSA®, bioMérieux, France). All the other isolates were subjected to Gram staining and subsequent preliminary identification tests (catalase, oxidase and motility) and then, depending on the first indications obtained, seeded in further culture media: MacConkey; Columbia CNA, Pseudomonas Agar Base, Sabouraud and chromogenic media selective for the KES group (Klebsiella, Enterobacter, Serratia). After isolation in purity, the strains were identified with the use of biochemical/enzyme galleries (API®-bioMérieux, France). To evaluate antibiotic resistance, the bacterium was subjected to the agar diffusion test (Kirby-Bauer method) using Muller Hinton agar (Oxoid®) according to the CLSI indications and classified, depending on the measurement of the inhibition halo diameter, as sensitive, resistant or intermediate according to the protocol of the National Committee on Clinical Laboratory Standards, on the basis of the manufacturer's instructions (Oxoid®, Ati, BD). Nine of the antibiotics most frequently used to treat otitis in veterinary medicine were tested simultaneously.

SPSS software (version 19; SPSS Inc., Chicago, IL, USA) was used for the statistical analysis.

### Summary of results obtained in 7 cases

**Table 1. Animal data and clinical diagnosis**

| Case | Breed | Gender | Age | Clinical diagnosis |
|---|---|---|---|---|
| 1 | French bulldog | F | 8a | Recurrent monolateral ceruminous otitis with stenosis |
| 2 | Mongrel | F | 4a | Recurrent bilateral ceruminous otitis |
| 3 | Cocker spaniel | F | 5a | Recurrent monolateral erythematous otitis |
| 4 | Mongrel | F | 5a | Recurrent monolateral erythematous otitis |
| 5 | Mongrel | F | 13a | Recurrent bilateral erythematous ceruminous otitis with stenosis |
| 6 | Fox terrier | M | 7a | Recurrent bilateral erythematous ceruminous otitis |
| 7 | German shepherd | M | 5a | Recurrent monolateral erythematous ceruminous otitis |

**Table 2 Variation in CADESI scores**

| Case | T0 | T7 | T14 | T21 |
|---|---|---|---|---|
| 1 | 4 | 3 | 2 | 2 (without treatment) |
| 2 | 0 | 0 | 0 | 0 |
| 3 | 11 | 3 | 4 | 3 |
| 4 | 10 | 2 | 2 | 2 |
| 5 | 42 | 24 | 21 | 21 |
| 6 | 10 | 6 | 6 | N/R |
| 7 | 17 | 6 | 6 | N/R |

**Table 3 Variation in EAM grading**

| Case | T0 | T7 | T14 | T21 |
|---|---|---|---|---|
| 1 | 2 | 3 | 2 | 1 |
| 2 | 4 | 0 | 0 | 0 |
| 3 | 4 | 7 | 4 | 0 |
| 4 | 12 | 6 | 4 | 4 |
| 5 | 28 | 16 | 16 | 16 |
| 6 | 16 | 2 | 2 | N/R |
| 7 | 10 | 10 | 8 | N/R |

**Table 4 Variation in cytological examination of material taken from EAM**

| Case | T0 | T7 | T14 | T21 |
|---|---|---|---|---|
| 1 | Corneocytes > 20 HPF | < 5 HPF | 5-10 HPF | 10-20 HPF |
| | Cocci/rods, 20-30 HPF (Proteus vulgaris present) | 10-20 HPF (Proteus absent) | 10-20 HPF | 20-30 HPF (S. interm) |
| 2 | Corneocytes 10-20 HPF | < 5 HPF | < 5 HPF | < 5 HPF |
| | Cocci/rods 20-30 HPF | None | None | None |
| | Malassezia 10-20 HPF | None | None | None |
| 3 | Corneocytes 5-10 HPF | < 5 HPF | None | < 5 HPF |
| | Cocci and Malassezia 10-20 HPF | 10-20 HPF | None | None |
| 4 | Corneocytes 10-20 HPF | < 5 HPF | < 5 HPF | < 5 HPF |
| | Cocci and Malassezia 20-30 HPF | Cocci 10-20 HPF | None | None |
| 5 | Corneocytes 10-20 HPF | 10-20 HPF | < 5 HPF | < 5 HPF |
| | Cocci 20-30 HPF Rods 10-20 HPF | 10-20 HPF | 10-20 HPF | Cocci 10-20 HPF |
| 6 | Corneocytes 10-20 HPF | 5-10 HPF | 5-10 HPF | N/R |
| | Cocci/rods 20-30 HPF | Cocci 10-20 HPF | Cocci 10-20 HPF | N/R |
| 7 | Corneocytes 10-20 HPF | 5-10 HPF | 5-10 HPF | N/R |
| | Cocci 30-40 HPF | 20-30 HPF | 20-30 HPF | N/R |
| | Rods 10-20 HPF | 10-20 HPF | None | |
| | Malassezia 10-20 HPF | 10-20 HPF | None | |

The clinical trial confirmed the efficacy of the composition in the treatment of disorders caused by the micro-organisms investigated, including particularly resistant pathogens such as Proteus (case 1).

## Claims

1. Compositions comprising lactoferrin hydrolysate and verbascoside or extracts containing it.

2. Compositions according to claim 1 further comprising glycerophosphoinositol or salts thereof.

3. Compositions according to claim 1, or 2 wherein the extract containing verbascoside is an extract of *Olea europaea, Syringa vulgaris* or other genera, species and varieties or cultivars containing it.

4. Compositions according to one or more of claims 1 to 3 for topical administration.

5. Compositions according to claim 4 in the form of oil-in-water emulsions, water-in-oil emulsions, gel, spray, shampoo, solution, lotion, foam or mousse.

6. Compositions according to claim 4 or 5 comprising lactoferrin hydrolysate in concentrations ranging from 0.0005% to 15% by weight, glycerophosphoinositol or salts thereof, as a solution containing at least 4% L-α-glycero-phospho-D-myo-inositol choline, in concentrations ranging from 0.0020% to 10% by weight, and verbascoside or an extract containing it in concentrations ranging from 0.0020% to 15% by weight.

7. Compositions according to one or more of claims 1 to 3 for oral or parenteral administration.

8. Compositions according to claim 7 suitable for daily administration of the following doses, per 10 Kg of body weight:
- lactoferrin hydrolysate: 0.005 mg to 5 grams;
- glycerophosphoinositol (as a solution containing at least 4% L-α-glycero-phospho-D-myo-inositol choline): 0.005 mg to 5 grams;
- verbascoside: 0.005 mg to 5 grams.

9. Compositions according to claim 7 or 8 in the form of lozenges, tablets, powders or granulates.

10. Compositions according to one or more of claims 1 to 9 for use in the veterinary, cosmetic and nutraceutical fields and in medical devices.

11. Compositions according to one or more of claims 1 to 9 for the treatment of erythematous and ceruminous otitis externa, for the dermatological treatment of skin infections of microbial origin, and for stomatological treatment of inflammations of the oral cavity, particularly those located in the gums (gingivitis, periodontitis), in cats and dogs.

## Patentansprüche

1. Zusammensetzungen, umfassend Lactoferrinhydrolysat und Verbascosid oder Extrakte, die es enthalten.

2. Zusammensetzungen gemäß Anspruch 1, die außerdem Glycerophosphoinositol oder Salze davon umfassen.

3. Zusammensetzungen gemäß Anspruch 1 oder 2, wobei der Extrakt, der Verbascosid enthält, ein Extrakt von *Olea europaea, Syringa vulgaris* oder anderen Gattungen, Spezies und Varietäten oder Kultivaren, die es enthalten, ist.

4. Zusammensetzungen gemäß einem oder mehreren der Ansprüche 1 bis 3 zur topischen Verabreichung.

5. Zusammensetzungen gemäß Anspruch 4 in der Form von Öl-in-Wasser-Emulsionen, Wasser-in-Öl-Emulsionen, Gel, Spray, Shampoo, Lösung, Lotion, Schaum oder Mousse.

6. Zusammensetzungen gemäß Anspruch 4 oder 5, umfassend Lactoferrinhydrolysat in Konzentrationen im Bereich von 0,0005 Gew.-% bis 15 Gew.-%, Glycerophosphoinositol oder Salze davon als Lösung, die wenigstens 4% L-α-Glycero-phospho-D-myo-inositolcholin enthält, in Konzentrationen im Bereich von 0,0020 Gew.-% bis 10 Gew.-% und Verbascosid oder einen Extrakt, der es enthält, in Konzentrationen im Bereich von 0,0020 Gew.-% bis 15 Gew.-%.

7. Zusammensetzungen gemäß einem oder mehreren der Ansprüche 1 bis 3 zur oralen oder parenteralen Verabreichung.

8. Zusammensetzungen gemäß Anspruch 7, die zur täglichen Verabreichung der folgenden Dosen pro 10 kg Körpergewicht geeignet sind:
- Lactoferrinhydrolysat: 0,005 mg bis 5 Gramm;
- Glycerophosphoinositol (als Lösung, die wenigstens 4% L-α-Glycero-phospho-D-myo-inositolcholin enthält): 0,005 mg bis 5 Gramm;
- Verbascosid: 0,005 mg bis 5 Gramm.

9. Zusammensetzungen gemäß Anspruch 7 oder 8 in der Form von Pastillen, Tabletten, Pulvern oder Granulat.

10. Zusammensetzungen gemäß einem oder mehreren der Ansprüche 1 bis 9 zur Verwendung in den Gebieten der Veterinärmedizin, der Kosmetik und der Nutraceuticals sowie in medizinischen Vorrichtungen.

11. Zusammensetzung gemäß einem oder mehreren der Ansprüche 1 bis 9 für die Behandlung von erythematöser und zeruminöser Otitis externa, für die dermatologische Behandlung von Hautinfektionen mikrobiellen Ursprungs und zur stomatologischen Behandlung von Entzündungen der Mundhöhle, insbesondere von solchen, die im Zahnfleisch lokalisiert sind, (Gingivitis, Periodontitis), bei Katzen und Hunden.

## Revendications

1. Compositions comprenant de l'hydrolysat de lactoferrine et du verbascoside ou des extraits le contenant.

2. Compositions selon la revendication 1 comprenant en outre du glycérophosphoinositol ou ses sels.

3. Compositions selon la revendication 1 ou 2 dans lesquelles l'extrait contenant le verbascoside est un extrait d'*Olea europaea,* de *Syringa vulgaris* ou d'un autre genre, espèce, et variétés ou cultivars le contenant.

4. Compositions selon l'une ou plusieurs des revendications 1 à 3 pour une administration topique.

5. Compositions selon la revendication 4 sous forme d'émulsions huile-dans-eau, d'émulsions eau-dans-huile, de gel, de spray, de shampoing, de solution, de lotion, d'écume ou de mousse.

6. Compositions selon la revendication 4 ou 5 comprenant de l'hydrolysat de lactoferrine aux concentrations se situant dans la plage allant de 0,0005 % à 15 % en poids, du glycérophosphoinositol ou ses sels, en solution contenant au moins 4 % de L-α-glycéro-phospho-D-myo-inositol choline, aux concentrations se situant dans la plage allant de 0,0020 % à 10 % en poids et du verbascoside ou un extrait le contenant aux concentrations se situant dans la plage allant de 0,0020 % à 15 % en poids.

7. Compositions selon l'une ou plusieurs des revendications 1 à 3 pour une administration orale ou parentérale.

8. Compositions selon la revendication 7 appropriées pour une administration quotidienne des doses suivantes, par 10 Kg de poids corporel :
- hydrolysat de lactoferrine : 0,005 mg à 5 grammes ;
- glycérophosphoinositol (en solution contenant au moins 4 % de L-α-glycéro-phospho-D-myo-inositol choline) : 0,005 mg à 5 grammes ;
- verbascoside : 0,005 mg à 5 grammes.

9. Compositions selon la revendication 7 ou 8 sous forme de pastilles, de comprimés, de poudres ou de granulés.

10. Compositions selon l'une ou plusieurs des revendications 1 à 9 pour une utilisation dans les domaines vétérinaire, cosmétique et nutraceutique et dans les dispositifs médicaux.

11. Compositions selon l'une ou plusieurs des revendications 1 à 9 pour le traitement de l'otite externe érythémateuse et cérumineuse, pour le traitement dermatologique des infections de la peau d'origine microbienne et pour le traitement stomatologique des inflammations de la cavité orale, en particulier celles situées dans les gommes (gingivite, périodontite) chez les chats et les chiens.
